(19) **Europäisches Patentamt / European Patent Office / Office européen des brevets**

(11) **EP 4 225 087 B1**

(12) ## EUROPEAN PATENT SPECIFICATION

(45) Date of publication and mention of the grant of the patent:
**19.11.2025 Bulletin 2025/47**

(21) Application number: **21786556.7**

(22) Date of filing: **08.10.2021**

(51) International Patent Classification (IPC):
**A24F 40/48** (2020.01)    **A24F 40/50** (2020.01)
**A61M 11/04** (2006.01)    **A61M 15/00** (2006.01)
**A61M 15/06** (2006.01)    A24F 40/30 (2020.01)
**A24F 40/10** (2020.01)

(52) Cooperative Patent Classification (CPC):
**A24F 40/48; A24F 40/50; A61M 11/042;**
**A61M 15/0003; A61M 15/06;** A24F 40/10;
A24F 40/30; A61M 15/0015; A61M 15/0016;
A61M 15/0086; A61M 2016/0027; A61M 2205/18;
A61M 2205/3331; A61M 2205/3368;
A61M 2205/3386;                              (Cont.)

(86) International application number:
**PCT/GB2021/052606**

(87) International publication number:
**WO 2022/074395 (14.04.2022 Gazette 2022/15)**

(54) **A DEVICE AND A METHOD FOR VAPORISING A VOLATILE MATERIAL**

VORRICHTUNG UND VERFAHREN ZUR VERDAMPFUNG EINES FLÜCHTIGEN MATERIALS

DISPOSITIF ET PROCÉDÉ POUR VAPORISER UN MATÉRIAU VOLATIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2020 GB 202016020**

(43) Date of publication of application:
**16.08.2023 Bulletin 2023/33**

(73) Proprietor: **Inflowvent Ltd.**
**Glasgow G66 7HF (GB)**

(72) Inventor: **STABLER, Peter Joseph**
**Glasgow G66 7HF (GB)**

(74) Representative: **Potter Clarkson**
**Chapel Quarter**
**Mount Street**
**Nottingham NG1 6HQ (GB)**

(56) References cited:
**WO-A1-2015/037925    US-A1- 2019 099 581**
**US-A1- 2019 358 416**

EP 4 225 087 B1

(52) Cooperative Patent Classification (CPC): (Cont.)
A61M 2205/3633; A61M 2205/3653;
A61M 2205/583; A61M 2205/588; A61M 2205/8206

**Description**

**BACKGROUND**

The present invention relates to a device for, and a method of, vaporising a volatile material and providing a known quantity of the volatile material in vapour form. The invention may have particular application, although not exclusively, in the field of medicinal and recreational inhalation devices as it may facilitate a person using the device to inhale a known dose of a particular material.

In this context, a volatile material is to be considered as a material that may be volatilised to form a vapour.

The dynamics of vaporisation exhibited in known devices for vaporising volatile materials, referred to herein as vaporisation devices, are driven by factors affecting diffusion and evaporation which are very variable and difficult, if not impossible, to predict accurately. The variability and unpredictability of known vaporisation devices places a number of limitations on their use, some examples of which are set out below.

Known vaporisation devices may rely on temperature monitoring to control the power supply to a heater forming part of the vaporisation device. However, it is very difficult to measure an average temperature accurately as temperature can fluctuate significantly within a system. Accordingly, it may be very difficult to control the power supply to the heater in a way that accurately and consistently maintains a desired average temperature in the system.

As well as being difficult to control temperature in known vaporisation devices, the temperature of the volatile material in either the liquid/solid phase or vapour phase cannot be easily predicted. This may be particularly disadvantageous in applications where there is a critical temperature required to obtain a desired product, such as in the decarboxylation reactions of herbal cannabis.

It may also be difficult to predict or control the rate of evaporation within known vaporisation devices. Therefore, the amount of volatile material received by a user inhaling the resulting vapour will be variable and imprecise. This may be detrimental if the volatile material is, or comprises, an active component which might be harmful if too much is consumed.

When vaporising a volatile material comprising more than one component with known vaporisation devices, there may be a tendency to fractionate different components so that the user will receive poorly controlled ratios of the components. Again, this may affect the dose of an active component that the user receives. Additionally, it may be difficult, or impossible, to know that a particular component has been depleted and, therefore, that any benefit provided by that component has been lost.

Known vaporisation devices may require the entire quantity of gas/vapour/mist inhaled by the user to be heated to the same temperature, this means that the user may inhale high temperature substances, which can be uncomfortable, and that a large amount of energy is required, which is inefficient and may necessitate large battery capacity and power.

Lastly, known vaporisation devices may require the action of a user's inhalation to vaporise the volatile material which necessitates active participation of the user. However, such active participation may not be possible if the user is incapacitated.

United States patent application US 2019/099581 A1 describes methods and systems for delivering anaesthetic agent to a patient. In one embodiment, a liquid anaesthetic agent container includes a base region, an interior of the base region configured to hold liquid anaesthetic agent, an adapter region, and a capillary force vaporizer (CFV) housed in the adapter region. The adapter region includes a coupling end configured to couple to a patient breathing circuit to supply anaesthetic agent vaporized by the CFV to a patient.

**SUMMARY OF THE INVENTION**

According to a first aspect of the invention there is provided a vaporisation device comprising a pressure chamber, a controller and a pressure sensor for measuring an internal pressure within the pressure chamber. The pressure chamber comprises a reservoir of volatile material, a heater electrically coupled to the controller and a choked flow outlet for allowing vapour to exit the pressure chamber under choked flow conditions. The controller is configured to control the heater in dependence on the measured internal pressure to cause vaporisation of the volatile material for the internal pressure to be sufficiently high that, in use, vapour exiting the pressure chamber through the choked flow outlet does so under choked flow conditions.

In use, when vapour exits the pressure chamber under choked flow conditions, the pressure chamber may be considered as a closed system. In other words, the conditions inside the pressure chamber are independent of those outside the pressure chamber. This means that by knowing some of the variables inside the pressure chamber, other variables such as the concentration of volatile material in the vapour phase may be known, or at least predicted accurately.

Accordingly, by means of the invention, a volatile material may be vaporised to form a volatile material vapour with a known, or predictable, concentration that may flow from the pressure chamber with a known, or predictable, mass flow rate. A user of the vaporisation device may therefore be able inhale a known quantity, or dose, of volatile material.

A vaporisation device according to the invention may therefore be advantageous over known vaporisation devices for which it is very difficult, if not impossible, to accurately predict what dose of volatile material a user might inhale with each inhalation.

In embodiments of the invention, the vaporisation device may further comprise a temperature sensor for measuring an internal temperature within the pressure chamber. Also, the controller may be configured to trigger a temperature alert if the measured internal temperature is indicative of the reservoir of volatile material being depleted. For example, the reservoir of volatile material may be depleted if the measured internal temperature is detected to increase above an expected temperature dependent on the measured internal pressure.

Such embodiments of the invention may therefore provide a user of the vaporisation device with a warning that he or she should replace or refill the reservoir of volatile material to ensure that the device is operating optimally and that the user may continue to inhale a consistent, known dose of the volatile material.

In embodiments of the invention, the volatile material comprises a plurality of volatile material components. In some such embodiments, at least one of the volatile material components may be a component with an associated sensory impact, such as a distinctive scent. This may be advantageous as when another component becomes depleted during use of the vaporisation device, the concentration of the component with a sensory impact will increase in order that the equilibrium in the closed system is maintained. The associated sensory impact of that component may accordingly increase in intensity and indicate to the user that a component has depleted, and that the reservoir of volatile material requires replacing or refilling.

In embodiments of the invention, the plurality of volatile material components may be immiscible, and the controller may be configured to cause a predetermined ratio of volatile material component concentrations in the vapour phase by controlling the heater. In particular, the heater may be controlled to cause vaporisation of the volatile material so that the internal pressure is maintained at a predetermined pressure that facilitates consistent and desirable concentrations of each volatile material component.

In other embodiments of the invention, the plurality of volatile material components may be miscible and deviate from Raoult's Law to form an azeotrope. In such embodiments, the controller may similarly be configured to cause a predetermined ratio of volatile material component concentrations in the vapour phase by controlling the heater. However, in these embodiments, the heater may be controlled to cause vaporisation of the volatile material so that an internal temperature is achieved (by manipulating the internal pressure) that takes advantage of the azeotropic characteristics of the volatile mixture to provide a known concentration of the its components.

In further embodiments of the invention, the volatile material comprises a plurality of volatile material components which are miscible and do not deviate from Raoult's Law. Some embodiments of the invention may additionally comprise a temperature display coupled to the temperature sensor and configured to indicate the measured internal temperature. By being able to monitor the temperature inside the pressure chamber, a user may be able to predict the ratio of volatile material component concentrations in the vapour phase according to Raoult's Law and therefore predict the dose of one or more active volatile material components that may be inhaled.

In embodiments of the invention, the vaporisation device may comprise a valve movable between an open configuration and a closed configuration such that when the valve is in the open position vapour is able to discharge from the pressure chamber through the choked flow outlet, and when the valve is in the closed position vapour is prevented from discharging from the pressure chamber through the choked flow outlet. The valve may be an internal valve forming part of the pressure chamber or an external valve forming part of an outlet chamber fluidly connected to the pressure chamber via the choked flow outlet.

By means of such embodiments, a user of the vaporisation device may choose when to allow flow of vapour from the pressure chamber. This may prevent the volatile material from being wasted and reduce the amount that the heater must be used to maintain the choked flow conditions (or any other desirable conditions) within the pressure chamber.

In embodiments of the invention, the vaporisation device may comprise a plurality of pressure chambers. This may allow different conditions of pressure and temperature to be maintained in each pressure chamber so that different volatile materials may be vaporised at conditions optimal for each material or in order to achieve a desired combination of different materials that might not be possible in a single pressure chamber.

According to a second aspect of the invention there is provided a method for vaporising a volatile material within a pressure chamber comprising a reservoir of volatile material, a heater and a choked flow outlet, comprising the steps of: measuring an internal pressure within the pressure chamber; and, controlling the heater in dependence on the measured internal pressure to cause vaporisation of the volatile material for the internal pressure to be sufficiently high such that vapour exiting the pressure chamber through the choked flow outlet does so under choked flow conditions.

**BRIEF DESCRIPTION OF THE DRAWINGS**

Examples of the present invention will now be described in detail with reference to the accompanying drawings, in which:

Figure 1 is a schematic representation of a vaporisation device according to an embodiment of the invention;

Figure 2 is a graphical representation of a phase boundary in a closed system;

Figure 3 is a graphical representation of isobaric flow through an outlet in choked flow conditions;

Figure 4 is a schematic representation of a vaporisation device according to an embodiment of the invention comprising a secondary chamber;

Figure 5 is a schematic representation of vaporisation device according to an embodiment of the invention comprising a plurality of pressure chambers;

Figure 6 is a schematic representation of vaporisation device according to an embodiment of the invention comprising an internal valve;

Figure 7 is a schematic representation of vaporisation device according to an embodiment of the invention comprising an external valve;

Figure 8 is a graphical representation of a phase boundary of a single component system and a phase boundary of a binary component system;

Figure 9 is a graphical representation of the variation of volatile material component concentrations through a dose cycle;

Figure 10 is a graphical representation of equilibrium vapour and liquid compositions of an ideal mixture at constant pressure; and,

Figure 11 is a schematic representation of vaporisation device according to an embodiment of the invention comprising a secondary chamber with a mouthpiece.

## DETAILED DESCRIPTION

Choked flow is where the velocity of vapour through an orifice approaches the speed of sound. Under such conditions, pressure, temperature and vapour density (collectively referred to herein as the atmosphere) upstream of the orifice are independent of the atmosphere downstream of the orifice.

Assuming ideal gas behaviour, steady-state choked flow may occur when the ratio between downstream pressure and upstream falls below a predictable value in accordance with Equation 1:

*Equation 1*

$$\frac{P_d}{P_{crit}} = \left(\frac{2}{\gamma + 1}\right)^{\frac{\gamma}{\gamma - 1}}$$

*Where:*

$P_d$ = *Pressure downstream of the orifice*

$P_{crit}$ = *Critical upstream pressure, above which choked flow conditions exist*

$\gamma$ = *heat capacity ratio* = $C_p/C_v$ *where:*

$C_P$ = *heat capacity at constant pressure*

$C_V$ = *heat capacity at constant volume*

Assuming that the maximum pressure downstream of the orifice is atmospheric pressure, the minimum upstream pressure that must be maintained in order to remain in choked flow condition may be calculated. Using water/steam as an example, the ratio of specific heats ($C_p/C_v$) is 1.33, therefore the critical pressure that must be maintained upstream of the orifice would be ≥188 kPa (abs).

In embodiments of the present invention, a vaporisation device having a pressure chamber is provided, the pressure chamber including a choked flow outlet suitable for allowing discharge of vapour from the pressure chamber under choked flow conditions. The pressure chamber houses a reservoir of volatile material and a heater that may introduce energy into the pressure chamber. The quantum of energy introduced may cause a proportion of the volatile material in the reservoir of volatile material to vaporise, thereby increasing the pressure and density of volatile material in the vapour phase by an amount relative to the quantum of energy.

The vaporisation device is provided with a pressure sensor and a controller to monitor pressure within the pressure chamber and control the heater, to ensure that pressure is maintained above the critical pressure required for choked flow conditions to exist as vapour exits the pressure chamber through the choked flow outlet.

In use, when choked flow conditions are present, the atmosphere within the pressure chamber may be considered as independent to the atmosphere outside of the pressure chamber. Therefore, the pressure chamber may be considered as a closed system.

By facilitating vaporisation of a volatile material in a closed system, the present invention may provide a wide range of advantage over known vaporisation devices due to the significantly improved control and predictability possible for a closed system in comparison to an open system. Examples of such advantages are described below with reference to the drawings.

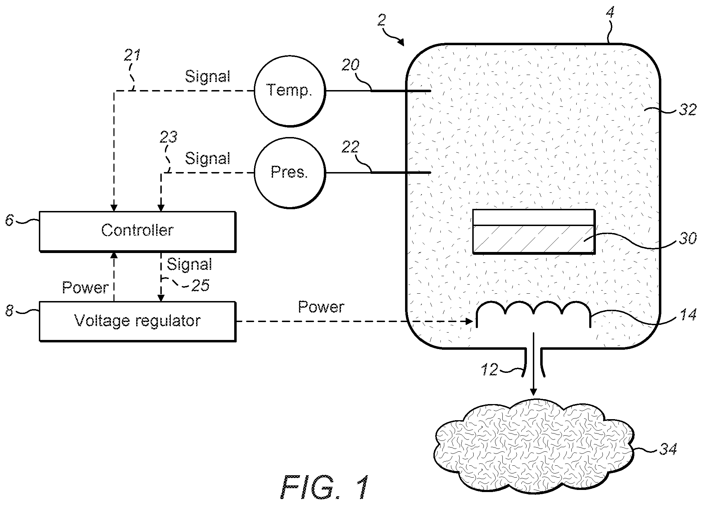

In Figure 1, a vaporisation device 2 according to an embodiment of the invention comprises a pressure chamber 4, a controller 6 and a voltage regulator 8. The pressure chamber 4 comprises a choked flow outlet 12, a heater 14 and a reservoir of volatile material 30 in a liquid or solid phase. In use, the volatile material may be vaporised to form a volatile material vapour 32 which may pass through the choked flow outlet 12, at which point the volatile material vapour 32 may condense and form a volatile material mist 34.

The volatile material may be any suitable volatile material. That is, any volatile material with a sufficient vapour *pressure*, under conditions of temperature and pressure suitable for a given application (such as a hand-held vaporised device), that may provide a required dose in terms of concentration of the volatile material, or a component of the volatile material, in the vapour phase. In the following description, water is frequently used as an example of a volatile material, but the invention is not limited to use with only water. Other volatile materials that may be vaporised advantageously by means of the invention include, but are not limited to, the following:

- terpenes such as limonene, geraniol, myrcene and menthol;
- organic solvents such as ether, chloroform, ethanol, naphtha and cresol;
- plant alkaloids such as cannabinoids, nicotine, caffeine, arecoline and guvacoline;
- pharmaceuticals, particularly (but not exlusively) free-bases; and
- essential oils.

Further, the volatile material may be a mixture of a plurality of volatile material components that may be miscible, immiscible or either depending upon the conditions.

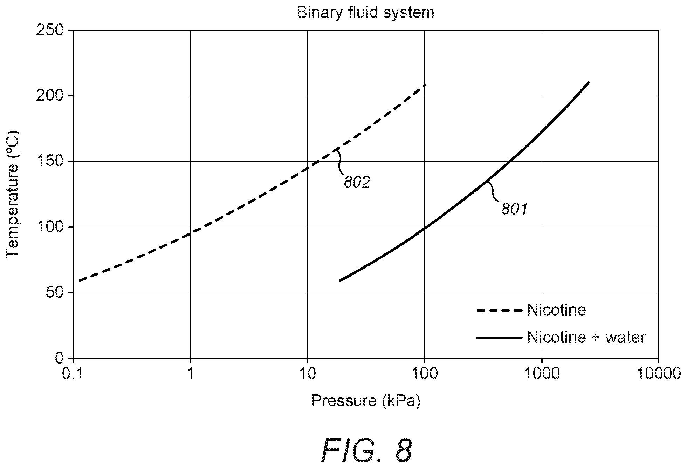

Use of such combinations of volatile materials is further described below, particularly with reference to Figures 8 to 12.

The vaporisation device 2 further comprises a temperature sensor 20 and a pressure sensor 22. The temperature sensor 20 may be configured to measure a temperature within the pressure chamber 4 and transmit a temperature signal 21 to the controller 6. Similarly, the pressure sensor 22 may be configured to measure pressure within the pressure chamber 4 and transmit a pressure signal 23 to the controller 6. The temperature and pressure signals 21, 23 may be any suitable type of signal to indicate the measured temperature or pressure. For example, each signal may be an electrical signal with a voltage proportional to the measured temperature or pressure.

The temperature signal 21 may be proportional to the temperature at the specific point in the pressurise chamber 4 where the temperature sensor 20 is located. It may not represent the overall temperature of the system, which may be affected by temperature gradients and may not be equal at all points within the system.

However, the pressure signal 23 may be proportional to the equilibrium pressure throughout the pressure chamber 4 due to the physical phenomenon that pressure exerts its force equally at all points within a static system.

The controller 6 may be a microcontroller or any other device suitable for receiving temperature and pressure signals 21, 23 and transmitting a heating signal 25 to the heater 14 via the voltage regulator 8.

The voltage regulator 8 may receive power from a power source (not shown) and transmit power, at a voltage regulated as required for proper function of the controller, to the controller 6. Also, based on the heating signal 25 received from the controller 6, the voltage regulator 8 may transmit power, at a voltage regulated based on the heating signal 25, to the heater 14.

The heater 14 may be any type of heater suitable for heating the contents of the pressure chamber. In Figure 1, the heater 14 is schematically represented as a heating filament positioned within the pressure chamber 4. However, in other embodiments of the invention the heater may instead comprise a heating filament that is positioned to surround outer walls of the pressure chamber, for example.

The choked flow outlet 12 may be any suitably shaped and sized orifice to allow vapour to flow through it at a desired flow rate under choked flow conditions. Further, the choked flow outlet 12 may be a *de Laval* nozzle.

In a closed system, the behaviour of gasses and liquids may be exploited to achieve and maintain an equilibrium where known gas laws will apply. Accordingly (and assuming ideal gas behaviour) the Universal Gas Law (Equation 2) may be applied to the pressure chamber 4 when the vaporisation device is in use and choked flow conditions are present (i.e. the pressure within the pressure chamber 4 is above the critical pressure required for choked flow to exist). Where non-ideal gas behaviour exists, additional terms (such as the compressibility factor of a gas) may be included in the equation.

*Equation 2*

$$PV = nRT$$

Where:

P    = Absolute pressure (kPa)

V    = Volume of vapour space (litres)

n    = Quantity of material in the vapour phase (moles)

R    = Universal gas constant (J.mol$^{-1}$.K$^{-1}$)

T    = Absolute temperature (K)

Therefore, by knowing any three of the four variables (pressure, volume, quantity of volatile material in the vapour phase and temperature) within the pressure chamber 4 the fourth variable may be calculated. This is not true for an open system, such as those provided by known vaporisation devices, as an open system can never reach the equilibrium required. As a consequence, an open system may be described in terms of evaporation rate and diffusion, rather than gas laws, wherein any prediction of variables within the system may be very complex, if not impossible.

Under certain conditions of temperature, pressure and quantity, the volatile material within the pressure chamber 4 may exist in both a liquid/solid phase (within the reservoir of volatile material 30) and a vapour phase (as the volatile material vapour 32) simultaneously. At this phase boundary condition, the pressure within the pressure chamber 4 equals the vapour pressure of the volatile material vapour 32. Also, the concentration, or density, of volatile material in the volatile material vapour 32 is directly proportional to the pressure.

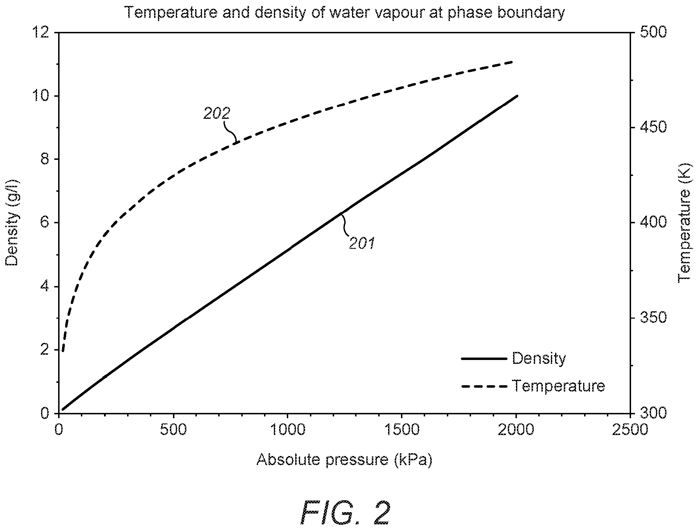

Figure 2 shows an example how the phase boundary conditions of water/steam may vary with absolute pressure within a closed system, such as that provided by the pressure chamber 4. Note that water/steam is not an ideal gas and an additional term 'Z', called the compressibility factor needs to be included in Equation 2.

In particular, the variation of density 201 with pressure, at the phase boundary, is a straight line and hence shows that the variation of density 201 is proportional to the variation of pressure (x axis). Meanwhile, the variation of temperature 202 is curved and hence not proportional to the variation of pressure.

Therefore, in a closed system, the density of volatile material vapour 32 may be calculated from a measurement of the pressure in the system (pressure chamber 4), without reference to temperature. Hence, due to the pressure chamber 4 forming a closed system under choked flow conditions, the invention may facilitate accurate calculation of the density of volatile material in the vapour phase. This may be particularly advantageous for accurately calculating a dose of an active volatile material to be inhaled by a user of the invention.

It is also possible to estimate the density of the volatile material in the vapour phase using temperature. However, the value may only be approximated using a derivation of the Clausius-Clapeyron equation or determined empirically by plotting log pressure against the inverse of temperature. Due to the increased complexity of such methods, this may be less convenient and less accurate than using pressure for the same purpose.

The mass flow rate of vapour that passes through the choked flow outlet 12 may be calculated according to Equation 3 wherein the mass flow rate is a function of the pressure within the pressure chamber 4, the area of the choked flow outlet 12 and the density of the volatile material vapour 32.

*Equation 3*

$$m = C_d A \sqrt{\rho_o P_o \gamma \left(\frac{2}{\gamma + 1}\right)^{\left(\frac{\gamma+1}{\gamma-1}\right)}}$$

Where:

m    = mass flow rate (kg.s$^{-1}$)
$C_d$    = Discharge coefficient, a function of outlet 12 geometry (dimensionless)
A    = choked flow outlet 12 minimum cross-sectional area (m$^2$)
$P_o$    = Pressure in the pressure chamber 4 (kPa)
$\rho_o$    = Vapour density at pressure $P_o$ (kg.m$^2$)

A desired mass flow rate of volatile material can therefore be achieved independently of the pressure outside of the pressure chamber 4 with any combination of choked flow outlet area / geometry and pressure within the pressure chamber 4, provided the pressure within the pressure chamber 4 is sufficiently great to maintain choked flow conditions.

In use, as volatile material vapour 32 is lost from the pressure chamber 4, through the choked flow outlet 12, it is replaced in the vapour phase by evaporation of volatile material in the solid/liquid phase present in the volatile material reservoir 30. The latent heat of vaporisation may be provided by the heater 14 as mentioned above. Advantageously, the heater 14 needs to provide only that power required to evaporate sufficient volatile material from the liquid/solid phase to replace that lost from the vapour phase via the choked flow outlet 12.

As long as two phases (the liquid/solid phase and the vapour phase) exist within the pressure chamber 4, the pressure, temperature and vapour phase density at the phase boundary equilibrium may be maintained by using the vaporisation device 2 in choked flow conditions and using the heater 14 to maintain a constant pressure within the pressure chamber 4.

However, this is no longer true if the system changes from two-phase to single-phase, i.e. once the reservoir of volatile material 30 is depleted (through vaporisation) and only the volatile material vapour 32 remains.

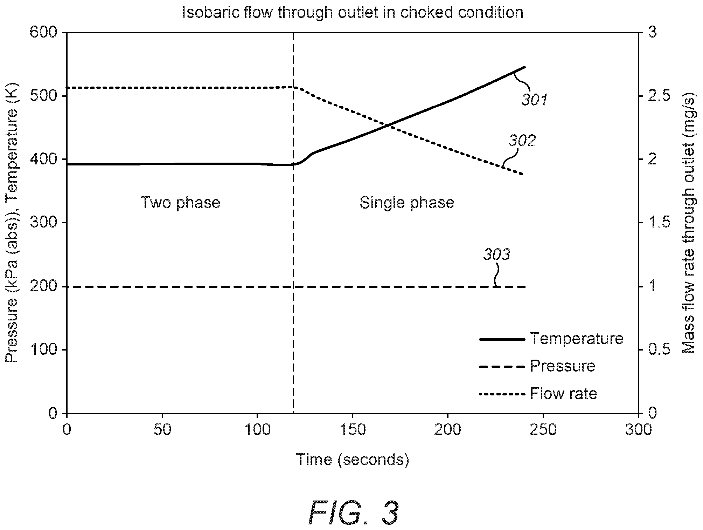

The effect of crossing the phase boundary of the volatile material - moving from a two-phase system (vapour phase and liquid/solid phase) to a single-phase system (vapour phase only) - can be observed in Figure 3. In particular, Figure 3 uses water again as an example volatile material and shows how temperature 301 in the pressure chamber, flow rate 302 through the choked flow outlet and pressure 303 in the pressure chamber each change over time.

For the first 120 seconds all variables remain constant. However, when the boundary condition is reached and the system changes from two phase to single phase, the temperature 301 increases. This may be expected from considering Equation 2 - the quantity of volatile material in the vapour phase ($n$) is reducing while the pressure ($P$) and volume ($V$) remain constant, hence temperature ($T$) must increase. Therefore, by monitoring for an increase in temperature, via the temperature sensor 20, it is possible to identify whether a change from a two-phase system to single-phase system occurs. In other words, by monitoring the temperature in the pressure chamber 4, it is possible to identify when the reservoir of volatile material 30 is depleted and needs replenishing.

The reservoir of volatile material 30 may be replenished by any suitable means. For example, in some embodiments of the invention the reservoir of volatile material 30 may be removable and either refillable once removed from the pressure chamber 4 or entirely replaceable with a full reservoir of volatile material. In further embodiments of the invention the reservoir of volatile material 30 may be integral with the pressure chamber 4 and may be refillable via a sealable inlet to the pressure chamber 4.

As shown in Figure 3, the mass flow rate through the outlet 302 decreases once the boundary condition is reached. This may be expected from considering Equation 3 - once the reservoir of volatile material 30 is depleted, the density of volatile material in the vapour phase ($\rho_o$) will begin decreasing and that reduction will be reflected in a reduction of the mass flow rate ($m$).

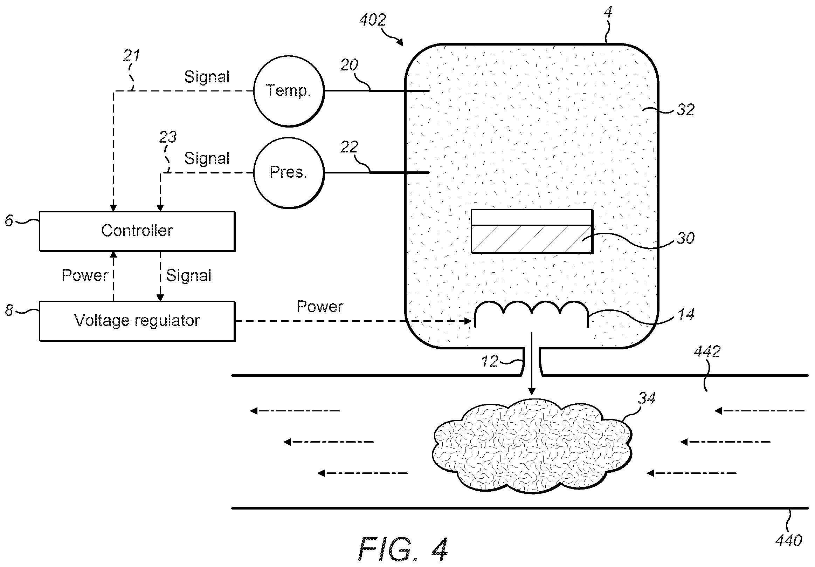

Referring now to Figure 4, a vaporisation device 402 is similar to the vaporisation device 2 shown in Figure 1. In addition to comprising the features shown in Figure 1 (provided with equivalent reference numerals), the vaporisation device 402 comprises a secondary chamber 440 wherein the choked flow outlet 12 extends from the pressure chamber 4 to the secondary chamber 440. A secondary gas 442 may flow through the secondary chamber 440, past the choked flow outlet 12 such that the volatile material mist 34 is mixed with the secondary gas 442 and carried through the secondary chamber 440 with the secondary gas 442.

In use, the secondary chamber 440 may facilitate delivery of the volatile material mist 34 to the user wherein the secondary gas 442 is air that flows from an inlet (not shown) to a mouthpiece (also not shown) through which the user may inhale the mixture of air 442 and volatile mixture mist 34.

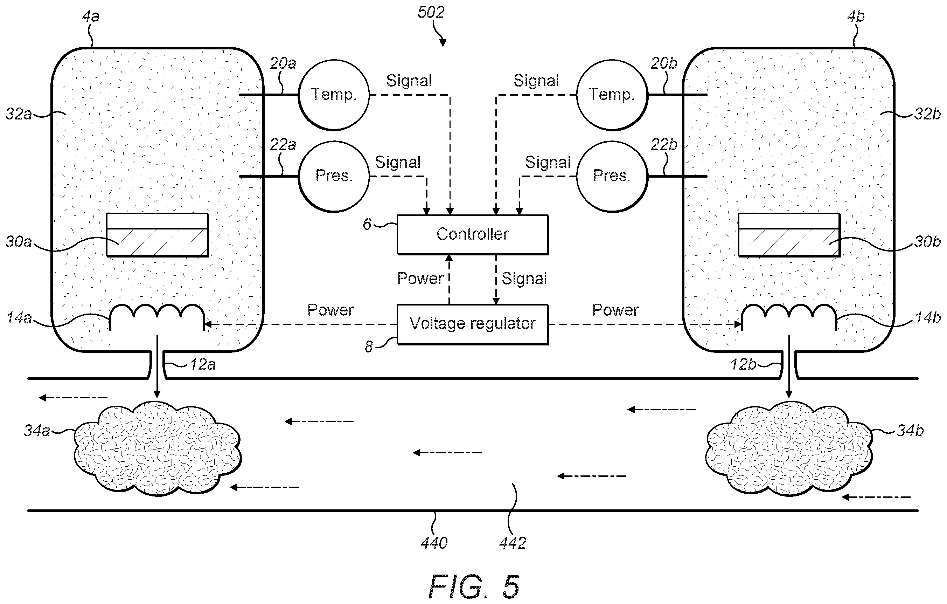

In Figure 5, a vaporisation device 502 is similar to the vaporisation device 402 shown in Figure 4 except that it comprises a first pressure chamber 4a and a second pressure chamber 4b, each with equivalent features to those shown in Figure 4 and annotated accordingly.

The vaporisation device 502 may facilitate the separate production of two different volatile material mists 34a, 34b that may be mixed together in the secondary gas 442 flowing through the secondary chamber 440 and delivered to a user. In one example, the vaporisation device 502 may allow for different conditions of pressure and temperature to be maintained in the separate pressure chambers 4a, 4b so that two different volatile materials may be vaporised at optimal conditions to provide the desired mass flow rate of each volatile material vapour 32a, 32b into the secondary chamber 440 to form volatile material mists 34a, 34b that may mix before being delivered to the user. In another example, the vaporisation device 502 may be used with the two pressure chambers 4a, 4b operating with the same conditions but different volatile materials that will only mix as condensates (volatile material mists 34a, 34b). This may be advantageous if the materials could interact unfavourably when together in their liquid or vapor phases or at increased temperatures and/or pressures, for example.

In other embodiments of the invention there may be any suitable number of pressure chambers (i.e. one, two or more than two). Selecting the number of pressure chambers to include may depend on a variety of factors including the end application (i.e. volatile material(s) to be vaporised), size and cost of the vaporisation device.

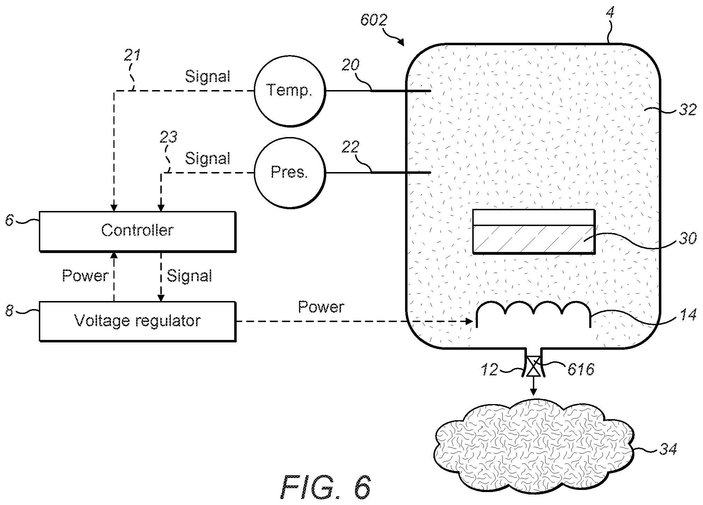

In Figure 6, another vaporisation device 602 is similar to the vaporisation device 2 shown in Figure 1. In addition to comprising the features shown in Figure 1 (provided with equivalent reference numerals), the pressure chamber 4 of the vaporisation device 602 comprises an internal valve 616 which may be integral with the choked flow outlet 12. The internal valve 616 may be movable between an open configuration and a closed configuration. When the internal valve 616 is in the open configuration, vapour may travel through the choked flow outlet 12 as described above. However, when the internal valve 616 is in the closed configuration, discharge of vapour from the pressure chamber 4 may be prevented. A user of the vaporisation device 602 may therefore choose when to allow vapour to flow from the pressure chamber 4 and when to interrupt that flow when it is not required.

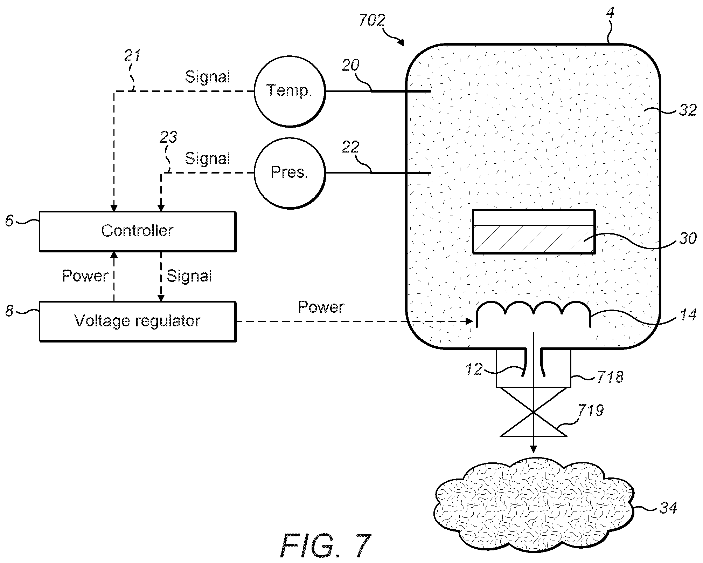

In Figure 7, a vaporisation device 702 is similar to the vaporisation device 602 shown in Figure 6 except that the vaporisation device 702 comprises an outlet chamber 718 coupled to the pressure chamber 4 and comprising an external valve 719, rather than the pressure chamber 4 comprising an internal valve. The external valve 719 may be fluidly connected to the choked flow outlet 12 via the outlet chamber 718.

Similarly to the internal valve 616, the external valve 719 may be movable between an open configuration and a closed configuration. When the external valve 719 is in the open configuration, vapour may travel through the choked flow outlet 12, through the outlet chamber 718 and out through external valve 719 where it may condense to form the volatile material mist 34. Conversely, when the external valve 719 is in the closed configuration, discharge of vapour from the outlet chamber 718 may be prevented. Volatile material vapour 32 may therefore pass through the choked flow outlet 12 until an equilibrium is reached between the pressure in the pressure chamber 12 and that in the outlet chamber 718, at which point the system may stabilise with the outlet chamber 718 essentially forming an extension of the pressure chamber 12 in terms of the conditions exhibited inside of it.

Up to this point, the invention has been described based on the pressure chamber (shown in any of Figures 1 and 4 to 7) being used to hold a single volatile material, with water as an example of that volatile material. However, a vaporisation device according to embodiments of the invention (such as those shown in Figures 1 and 4 to 7) may also be used to advantageously vaporise a volatile material comprising two or more volatile components.

A binary fluid system may be created by combining two or more volatile fluids or solids within a pressure chamber (from herein pressure chamber 402 shown in Figure 4 will be used as an example, although the same considerations may be applied to any pressure chamber according to embodiments of the invention). Daltons law of partial pressures states that the total pressure within an atmosphere is equal to the sum of the partial pressures of the component gasses at the temperature of the atmosphere within the system (pressure chamber 402).

<div align="center"><em>Equation 4</em></div>

$$P_{Total} = \sum_{i=1}^{n} P_i$$

*Where:*

$P_{Total}$     *= Total system pressure*
$P_i$          *= $P_i = P_{Total}.x_i$ where:*
$x_i$          *= mole fraction of the ith component of the total mixture of n components*

An example of a binary fluid system is one with a volatile material comprising the components nicotine and water, which are immiscible fluids between the temperatures of 60 °C and 210 °C. In other words, when nicotine and water are combined at temperatures between 60° and 210° it is not possible to mix them to form a homogenous substance. In Figure 8, the phase boundary of nicotine and water 801, within the immiscible temperature range, is shown in contrast with the phase boundary of nicotine only 802 (in a single fluid system, rather than a binary fluid system). In this example, adding water to nicotine facilitates the vaporisation of nicotine with temperatures needed to achieve choked flow conditions reduced by approximately 100 °C when compared with vaporising nicotine without water present.

Hence, due to the pressure chamber 4 providing a closed system, the invention may be used to advantageously exploit the property of binary fluids/solids within the pressure chamber 4 so that the temperature needed to attain choked flow conditions is changed compared to either of the constituent fluid/solid components in isolation.

The partial pressure of the vapours within immiscible binary fluid systems, such as nicotine and water system in temperatures within the immiscible range (60 °C to 210 °C), are independent of the quantity of volatile liquid or solid within the system provided none of the constituent components becomes depleted from the liquid or solid phases. In addition, the concentration of each volatile material component in the vapour phase is dependent on the temperature and specific to the particular component. Therefore, by means of the invention, the proportions of volatile material components in the vapour phase may be adjusted by selecting appropriate pressures equivalent to the required temperatures (to be controlled by the controller 6).

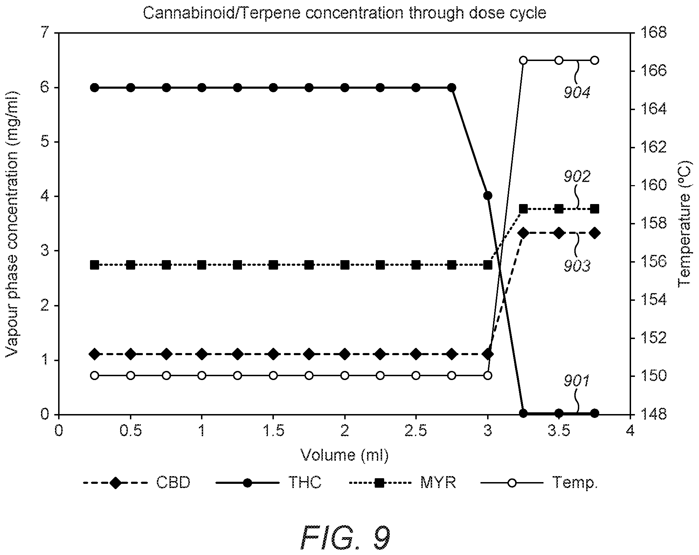

For example, in Figure 9, another binary fluid/solid system is shown wherein the volatile material is formed of immiscible components THC, CBD and Myrcene. The graph represents progression through a dose cycle and shows the variation of the vapour phase concentrations of THC 901, myrcene 902 and CBD 903 as volatile material vapour 32 exits the pressure chamber 4 (thereby providing a dose of increasing volume to the user, in this example). Initially, the vapour phase concentrations 901, 902, 903 remain constant despite the volatile material components in the liquid/solid form depleting at different rates from the reservoir of volatile material reservoir 30 (and therefore being present in varying ratios) throughout the dose cycle. This demonstrates the independence of the vapour phase concentration of each component from the quantity of each liquid or solid component within the system (provided no component is fully depleted, as mentioned above).

Therefore, by virtue of the behaviour of immiscible volatile materials in a closed system such as the pressure chamber, the invention may be used to provide a consistent dose of one or more volatile material components to the user regardless of the relative quantities of the volatile material components present in the liquid/solid phase. This characteristic may be particularly advantageous in allowing a user to reduce variation of the concentration of an active component between doses due to variability in the raw volatile material components present in the reservoir of volatile material.

Further, the invention may be used to advantageously exploit the property of immiscible volatile materials having different partial pressures at different temperatures by using temperature in the pressure chamber 4 (controlled via adjusting total pressure in the pressure chamber 4) to adjust and control the ratio of the volatile material components in the vapour phase and thus the composition of volatile material components aspirated by the user as the volatile material mist 34, for example.

Referring to Figure 9 again, after 2.75 ml of volatile material has exited the pressure chamber 4, the vapour phase concentration of THC 901 begins decreasing to 0 mg/ml. This is due to the THC in the liquid/solid phase being depleted from the reservoir of volatile material such that no further vapour can be produced to maintain the equilibrium. The depletion of THC causes an increase in the partial pressure of the remaining components as the temperature is increased to maintain a static pressure (as demonstrated by the variation of temperature 904 through the close cycle). In this example, one of the components that increases in vapour phase concentration is myrcene - a terpene with a pleasant clove-like aroma. The increase in the vapour phase concentration of myrcene 902 may be detected by the user due to an increase in strength of the associated clove-like aroma. A similar increase in the clove-like aroma may also occur if the CBD was depleted. Conversely, if the myrcene was depleted the user may notice a reduction in the clove-like aroma. Alternatively, the increase in temperature required to maintain a static pressure may also be used to indicate the depletion of one or more of the components.

Hence, by means of the present invention it is possible to use a volatile material comprising a component with an associated sensory impact, such as fragrance, to indicate the depletion of one or more of the components forming part of the volatile material.

Some binary solid/fluid systems comprise miscible components that mix together to form a homogeneous substance, such as nicotine and water at temperatures below 60° or above 210° for example.

Raoult's law predicts that the vapour pressure of a miscible mixture is equal to the weighted sum of the 'pure' vapour pressures of the components of the miscible mixture. Thus, the mixture vapour pressure for a mixture of two components, 'A' and 'B' may be given by the equation 5.

*Equation 5*

$$P = P_a x_a + P_b x_b$$

*Where:*

$P$ = Total vapour pressure
$P_a$ = *Vapour* pressure of component 'a'
$x_a$ = Mol fraction of component 'a' (liquid/solid)
$P_b$ = *Vapour* pressure of component 'b'

Ideal mixtures of miscible volatile materials that do not deviate from Raoult's Law may not maintain a constant vapour phase composition, as may be the case with the mixture previously. Instead, when such a binary system is in equilibrium, at constant pressure, the vapour phase composition will be affected by the composition within the solid/liquid phase in a predictable manner that may be determined by monitoring the temperature.

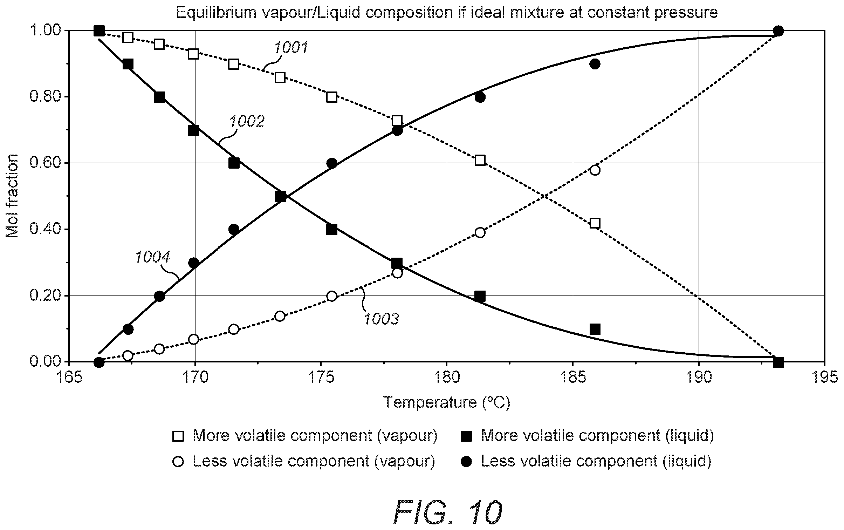

For example, Figure 10 shows how, at constant pressure, the mole fractions of the components of a binary system comprising non-azeotropic miscible volatile materials may vary with temperature in the system (and vice versa). The system comprises a more volatile component and a less volatile component. As temperature increases, the mole fraction of the more volatile component, in both the vapour phase 1001 and the liquid phase 1002, decreases while the mole fraction of the less volatile component, in both the vapour phase 1003 and the liquid phase 1004, increases.

By understanding this relationship and knowing the pressure and temperature in a closed system such as that provided by the pressure chamber of a vaporisation device according to embodiments of the invention (such as those shown in Figures 1 and 4 to 7), mole fractions of the vapour phase will be predictable. This can allow a user of the vaporisation device 2 to accurately estimate how much of each volatile material component exits the pressure chamber for inhalation, for example.

Binary solid/fluid systems can also be obtained from miscible volatile fluid or solid mixtures that have a positive or negative deviation from Raoult's Law forming an azeotrope.

An 'Azeotrope' is a vapour pressure at which the molar ratio of the constituent components in the vapour phase is identical to the molar-ratio of the constituent components in the miscible liquid/solid phase. Therefore, provided the azeotrope is maintained through controlling *pressure,* the composition of the vapour phase will be constant.

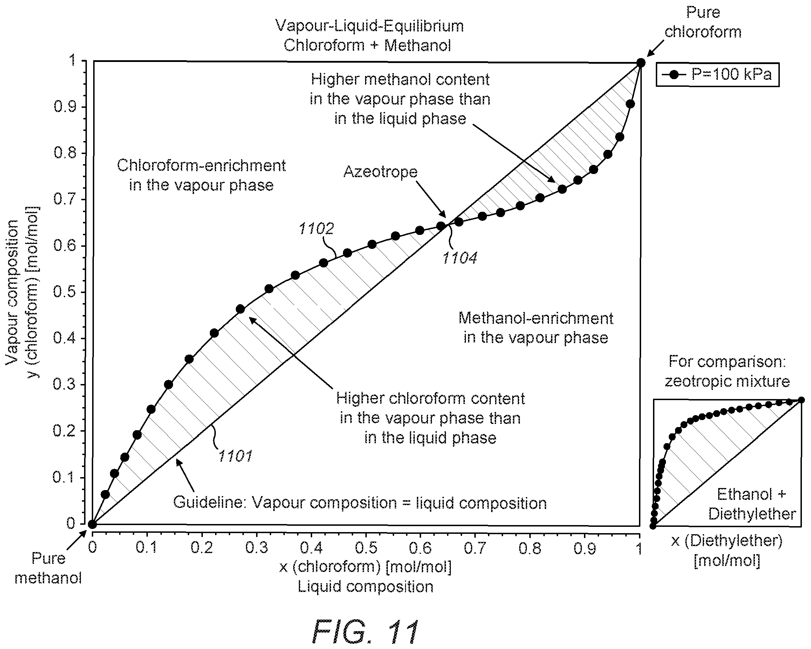

For example, Figure 11 shows a graph based on an example of a binary system comprising miscible volatile components - chloroform and methanol. A guideline 1101 indicates what would be an equal mole fraction of chloroform in the vapour phase (y-axis) and liquid phase (x-axis). However, a true variation in mole fractions of chloroform in the vapour and liquid phases 1102 (at constant pressure) strays from the guideline 1101. Only at an azeotrope 1104 are the mole fractions of chloroform 1102 equal in the vapour and liquid phases.

The composition of the azeotrope may be adjusted by selecting a suitable operating pressure. The actual pressure selected may be determined by experimentation.

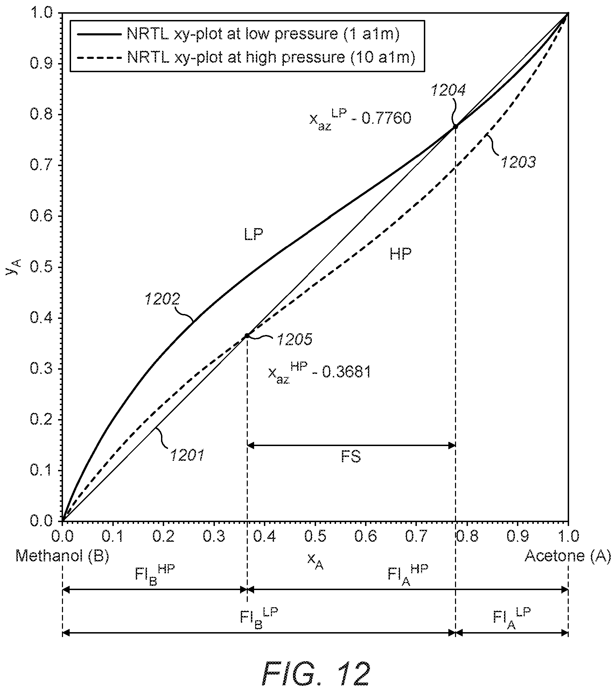

For example, Figure 12 shows a graph based on another example of a binary system, this system comprising the miscible volatile components acetone and methanol. Similarly to Figure 11, a guideline 1201 indicates what would be an equal mole fraction of acetone in the vapour phase (y-axis) and liquid phase (x-axis). In this instance, the variation in mole fractions of acetone in the vapour and liquid phases is shown at a low pressure 1202 (1 atm) and a high pressure 1203 (10 atm). A low-pressure azeotrope 1204 exists at acetone mole fractions of 0.7760 while a high-pressure azeotrope exists with acetone mole fractions of 0.3681.

A vaporisation device according to embodiments of the invention (such as those shown in Figures 1 and 4 to 7), may be used with knowledge of deviations from Raoult's Law to use an azeotrope of two (or more) volatile material components to deliver a consistent composition of the volatile material components to a user. Adjustment of the vapour pressure at which the pressure chamber 2 is maintained may be used to change the composition of the volatile material components in the mixture at which the azeotrope exists and thus the composition of volatile material components provided to the user in the volatile material mist 34.

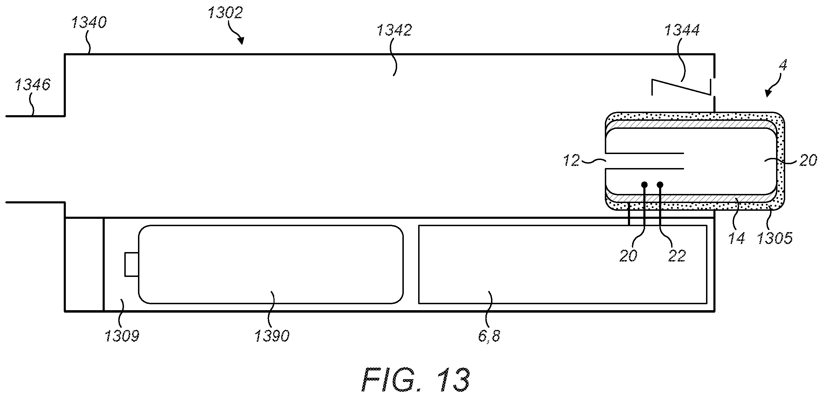

Referring now to Figure 13, a further embodiment of a vaporisation device 1302 according to the invention comprises a pressure chamber 4 similar to those shown in Figures 1 and 4 to 7 except, in this embodiment of the invention, the heater 14 is configured to surround the pressure chamber 4 and an insulating layer 15 surrounds both the heater 14 and the pressure chamber 4.

Further, the vaporisation device 1302 comprises a secondary chamber 1340 similar to the secondary chamber 440 forming part of the vaporisation device 402 (shown in Figure 4). In this embodiment of the invention, the secondary chamber 1340 comprises an inlet valve 1344 and a mouthpiece 1346. A secondary gas 1342, such as air, may flow into the secondary chamber 1340 via the inlet valve 1344, passed the choked flow outlet 12 of the pressure chamber 4 and out of the secondary chamber 1340 via the mouthpiece 1346. In use, a volatile material mist (not shown) produced from the pressure chamber 4 may mix with the secondary gas 1342 in the secondary chamber 1340 for inhalation by a user, via the mouthpiece 1346. The suction of the secondary gas 1342 and volatile material mist from the secondary chamber 1340 may cause a reduction of pressure within the secondary chamber 1340. The inlet valve 1344 may be configured so that, when there is a differential in pressure across the inlet valve 1344, it allows gas to travel through it until the upstream and downstream pressures are substantially in equilibrium. Accordingly, as the secondary gas and volatile material mist mixture is inhaled, further secondary gas 1342 may enter the secondary chamber 1340 to replace the inhaled mixture and mix with fresh volatile mixture for the next inhalation.

The vaporisation device 1302 also comprises a power source receptacle 1309 that may receive a power source 1390 such as a battery. In some embodiments of the invention the power source 1390 may be removable and may, therefore, be replaced once the energy stored within it is depleted. In other embodiments of the invention the vaporisation device 1302 may further comprise a charging socket (not shown) for receiving a charging cable such that the power source 1390 may be recharged as required by the user.

**Claims**

1. A vaporisation device (2) comprising a pressure chamber (4), a controller (6), and a pressure sensor for measuring an internal pressure within the pressure chamber, wherein:

   the pressure chamber comprises a reservoir (30) of volatile material, a heater (14) electrically coupled to the controller and a choked flow outlet (12) for allowing vapour (32) to exit the pressure chamber under choked flow conditions; and,

the controller is configured to control the heater in dependence on the measured internal pressure, **characterised in that** the controller is configured to control the heater to cause vaporisation of the volatile material for the internal pressure to be sufficiently high that, in use, vapour exiting the pressure chamber through the choked flow outlet does so under choked flow conditions.

2. A vaporisation device according to claim 1, wherein the controller (6) is adapted to control the heater (14) by providing a variable voltage to the heater.

3. A vaporisation device according to claim 1 or 2, further comprising a voltage regulator (8) electrically coupled to the controller (6), wherein the heater (14) is electrically coupled to the controller via the voltage regulator.

4. A vaporisation device according to any preceding claim, further comprising a temperature sensor (20) for measuring an internal temperature within the pressure chamber (4), wherein the controller (6) is configured to trigger a temperature alert if the measured internal temperature is indicative of the reservoir (30) of volatile material being depleted, and preferably, the controller is configured to trigger the temperature alert when the measured internal temperature is detected to increase above an expected temperature dependent on the measured internal pressure.

5. A vaporisation device according to any preceding claim, wherein the volatile material comprises a plurality of volatile material components, and preferably at least one of the volatile material components is a component with an associated sensory impact.

6. A vaporisation device according to claim 5, wherein the plurality of volatile material components are immiscible, and the controller (6) is configured to cause a predetermined ratio of vapour phase volatile material component concentrations by controlling the heater (14).

7. A vaporisation device according to claim 5, wherein the plurality of volatile material components are miscible and deviate from Raoult's Law, and the controller (6) is configured to cause a predetermined ratio of vapour phase volatile material component concentrations by controlling the heater (14).

8. A vaporisation device according to any preceding claim, further comprising a secondary chamber (440) fluidly connectable to the pressure chamber (4) via the choked flow outlet (12), and preferably the secondary chamber comprises an inlet valve (1344) through which gas may enter the secondary chamber and a mouthpiece (1346) through which fluid may exit the secondary chamber.

9. A vaporisation device according to any preceding claim, wherein the pressure chamber (4) comprises an internal valve (616) movable between an open configuration and a closed configuration, such that when the internal valve is in the open position vapour (32) is able to discharge from the pressure chamber through the choked flow outlet (12), and when the internal valve is in the closed position vapour is prevented from discharging from the pressure chamber through the choked flow outlet.

10. A vaporisation device according to any one of claims 1 to 8, further comprising an outlet chamber (718) fluidly connected to the pressure chamber (4) via the choked flow outlet (12), wherein the outlet chamber comprises an external valve (719) movable between an open configuration and a closed configuration, such that when the external valve is in the open position vapour is able to discharge from the outlet chamber through the external valve, and when the external valve is in the closed position vapour is prevented from discharging from the outlet chamber through the external valve.

11. A vaporisation device according to any preceding claim, wherein the reservoir (30) of volatile material is removable from the pressure chamber (4) and is replaceable or refillable, or wherein the reservoir of volatile material is integral with the pressure chamber and is refillable.

12. A method for vaporising a volatile material within a pressure chamber (4) comprising a reservoir (30) of volatile material, a heater (14) and a choked flow outlet (12), comprising the steps of:

measuring an internal pressure within the pressure chamber; and,
controlling the heater in dependence on the measured internal pressure, **characterised in that** the heater is controlled to cause vaporisation of the volatile material for the internal pressure to be sufficiently high such that vapour exiting the pressure chamber through the choked flow outlet does so under choked flow conditions.

13. A method according to claim 12, wherein the step of controlling the heater (14) comprises providing a variable voltage to the heater.

14. A method according to claim 12 or 13, wherein the volatile material comprises a plurality of volatile material components and the method further comprises the step of causing a predetermined ratio of vapour phase volatile material component concentrations by controlling the heater (14).

15. A method according to claim 14, wherein the plurality of volatile material components are immiscible, or wherein the plurality of volatile material components are miscible and deviate from Raoult's Law.

**Patentansprüche**

1. Verdampfungsvorrichtung (2) mit einer Druckkammer (4), einer Steuerung (6) und einem Drucksensor zum Messen eines Innendrucks innerhalb der Druckkammer, wobei:

   die Druckkammer einen Behälter (30) für flüchtiges Material, einen elektrisch mit der Steuerung gekoppelten Heizer (14) und einen gedrosselten Strömungsauslass (12) umfasst, damit Dampf (32) unter gedrosselten Strömungsbedingungen aus der Druckkammer austreten kann; und,
   die Steuerung konfiguriert ist, um den Heizer in Abhängigkeit vom gemessenen Innendruck zu steuern, **dadurch gekennzeichnet, dass** die Steuerung konfiguriert ist, um den Heizer so zu steuern, dass eine Verdampfung des flüchtigen Materials bewirkt wird, damit der Innendruck ausreichend hoch ist, dass bei Gebrauch der Dampf, der die Druckkammer durch den gedrosselten Strömungsauslass verlässt, dies unter gedrosselten Strömungsbedingungen tut.

2. Verdampfungsvorrichtung nach Anspruch 1, wobei die Steuerung (6) geeignet ist, den Heizer (14) durch Bereitstellen einer variablen Spannung für den Heizer zu steuern.

3. Verdampfungsvorrichtung nach Anspruch 1 oder 2, ferner umfassend einen Spannungsregler (8), der elektrisch mit der Steuerung (6) gekoppelt ist, wobei der Heizer (14) über den Spannungsregler elektrisch mit der Steuerung gekoppelt ist.

4. Verdampfungsvorrichtung nach einem der vorstehenden Ansprüche, ferner umfassend einen Temperatursensor (20) zum Messen einer Innentemperatur innerhalb der Druckkammer (4), wobei die Steuerung (6) konfiguriert ist, um einen Temperaturalarm auszulösen, wenn die gemessene Innentemperatur darauf hindeutet, dass das Reservoir (30) für flüchtiges Material erschöpft ist, und vorzugsweise die Steuerung konfiguriert ist, um den Temperaturalarm auszulösen, wenn festgestellt wird, dass die gemessene Innentemperatur über eine erwartete Temperatur ansteigt, die von dem gemessenen Innendruck abhängt.

5. Verdampfungsvorrichtung nach einem der vorstehenden Ansprüche, wobei das flüchtige Material eine Vielzahl von flüchtigen Materialkomponenten umfasst und vorzugsweise mindestens eine der flüchtigen Materialkomponenten eine Komponente mit einer damit verbundenen sensorischen Wirkung ist.

6. Verdampfungsvorrichtung nach Anspruch 5, wobei die Vielzahl flüchtiger Materialkomponenten nicht mischbar ist und die Steuerung (6) konfiguriert ist, um durch Steuern der Heizvorrichtung (14) ein vorbestimmtes Verhältnis der Konzentrationen der flüchtigen Materialkomponenten in der Dampfphase zu bewirken.

7. Verdampfungsvorrichtung nach Anspruch 5, wobei die Vielzahl flüchtiger Materialkomponenten mischbar ist und vom Raoultschen Gesetz abweicht und die Steuerung (6) konfiguriert ist, um durch Steuern des Heizers (14) ein vorbestimmtes Verhältnis der Konzentrationen der flüchtigen Materialkomponenten in der Dampfphase zu bewirken.

8. Verdampfungsvorrichtung nach einem der vorstehenden Ansprüche, ferner umfassend eine Sekundärkammer (440), die über den gedrosselten Strömungsauslass (12) fluidisch mit der Druckkammer (4) verbunden werden kann, wobei die Sekundärkammer vorzugsweise ein Einlassventil (1344), durch das Gas in die Sekundärkammer eintreten kann, und ein Mundstück (1346) umfasst, durch das Fluid aus der Sekundärkammer austreten kann.

## EP 4 225 087 B1

9. Verdampfungsvorrichtung nach einem der vorstehenden Ansprüche, wobei die Druckkammer (4) ein internes Ventil (616) umfasst, das zwischen einer offenen Konfiguration und einer geschlossenen Konfiguration beweglich ist, sodass, wenn sich das interne Ventil in der offenen Position befindet, Dampf (32) aus der Druckkammer durch den gedrosselten Strömungsauslass (12) abgelassen werden kann, und wenn sich das interne Ventil in der geschlossenen Position befindet, wird der Dampf daran gehindert, aus der Druckkammer durch den gedrosselten Strömungsauslass abgelassen zu werden.

10. Verdampfungsvorrichtung nach einem der Ansprüche 1 bis 8, ferner umfassend eine Auslasskammer (718), die über den gedrosselten Strömungsauslass (12) fluidisch mit der Druckkammer (4) verbunden ist, wobei die Auslasskammer ein externes Ventil (719) umfasst, das zwischen einer offenen Konfiguration und einer geschlossenen Konfiguration beweglich ist, sodass, wenn sich das externe Ventil in der offenen Position befindet, Dampf aus der Auslasskammer durch das externe Ventil abgelassen werden kann, und wenn sich das externe Ventil in der geschlossenen Position befindet, verhindert wird, dass Dampf aus der Auslasskammer durch das externe Ventil abgelassen wird.

11. Verdampfungsvorrichtung nach einem der vorstehenden Ansprüche, wobei der Behälter (30) für flüchtiges Material aus der Druckkammer (4) herausnehmbar und austauschbar oder nachfüllbar ist, oder wobei der Behälter für flüchtiges Material fest mit der Druckkammer verbunden und nachfüllbar ist.

12. Verfahren zum Verdampfen eines flüchtigen Materials in einer Druckkammer (4), die ein Reservoir (30) für flüchtiges Material, einen Heizer (14) und einen gedrosselten Strömungsauslass (12) umfasst, umfassend die folgenden Schritte:

   Messen eines Innendrucks innerhalb der Druckkammer; und,
   Steuern des Heizers in Abhängigkeit vom gemessenen Innendruck, **dadurch gekennzeichnet, dass** der Heizer gesteuert wird, um eine Verdampfung des flüchtigen Materials zu bewirken, damit der Innendruck ausreichend hoch ist, sodass der Dampf, der die Druckkammer durch den gedrosselten Strömungsauslass verlässt, dies unter gedrosselten Strömungsbedingungen tut.

13. Verfahren nach Anspruch 12, wobei der Schritt des Steuerns des Heizers (14) das Bereitstellen einer variablen Spannung für den Heizer umfasst.

14. Verfahren nach Anspruch 12 oder 13, wobei das flüchtige Material eine Vielzahl von flüchtigen Materialkomponenten umfasst und das Verfahren ferner den Schritt umfasst, durch Steuern des Heizers (14) ein vorbestimmtes Verhältnis der Konzentrationen der flüchtigen Materialkomponenten in der Dampfphase zu bewirken.

15. Verfahren nach Anspruch 14, wobei die Vielzahl flüchtiger Materialkomponenten nicht mischbar ist oder wobei die Vielzahl flüchtiger Materialkomponenten mischbar ist und vom Raoultschen Gesetz abweicht.

## Revendications

1. Dispositif de vaporisation (2) comprenant une chambre de pression (4), un dispositif de commande (6) et un capteur de pression pour mesurer une pression interne à l'intérieur de la chambre de pression, dans lequel :

   la chambre de pression comprend un réservoir (30) de matériau volatil, un dispositif de chauffage (14) couplé électriquement au dispositif de commande et une sortie d'écoulement obstruée (12) pour permettre à la vapeur (32) de sortir de la chambre de pression dans des conditions d'écoulement obstrué ; et,
   le dispositif de commande est configuré pour commander le dispositif de chauffage en fonction de la pression interne mesurée, **caractérisé en ce que** le dispositif de commande est configuré pour commander le dispositif de chauffage pour amener la vaporisation de matériau volatil pour que la pression interne soit suffisamment élevée pour que, lors de l'utilisation, la vapeur sortant de la chambre de pression par la sortie d'écoulement obstruée le fasse dans des conditions d'écoulement obstrué.

2. Dispositif de vaporisation selon la revendication 1, dans lequel le dispositif de commande (6) est conçu pour commander le dispositif de chauffage (14) en fournissant une tension variable au dispositif de chauffage.

3. Dispositif de vaporisation selon la revendication 1 ou 2, comprenant également un régulateur de tension (8) couplé électriquement au dispositif de commande (6), dans lequel le dispositif de chauffage (14) est couplé électriquement au dispositif de commande par le biais du régulateur de tension.

4. Dispositif de vaporisation selon une quelconque revendication précédente, comprenant également un capteur de température (20) pour mesurer une température interne à l'intérieur de la chambre de pression (4), dans lequel le dispositif de commande (6) est configuré pour déclencher une alerte de température si la température interne mesurée indique que le réservoir (30) de matériau volatil est épuisé, et de préférence, le dispositif de commande est configuré pour déclencher l'alerte de température lorsque la température interne mesurée est détectée comme augmentant au-dessus d'une température attendue en fonction de la pression interne mesurée.

5. Dispositif de vaporisation selon une quelconque revendication précédente, dans lequel le matériau volatil comprend une pluralité de composants de matériau volatil, et de préférence au moins l'un des composants de matériau volatil est un composant avec un impact sensoriel associé.

6. Dispositif de vaporisation selon la revendication 5, dans lequel la pluralité de composants de matériau volatil sont immiscibles, et le dispositif de commande (6) est configuré pour amener un rapport prédéterminé de concentrations de composants de matériau volatil en phase vapeur en commandant le dispositif de chauffage (14).

7. Dispositif de vaporisation selon la revendication 5, dans lequel la pluralité de composants de matériau volatil sont miscibles et s'écartent de la loi de Raoult, et le dispositif de commande (6) est configuré pour amener un rapport prédéterminé de concentrations de composants de matériau volatil en phase vapeur en commandant le dispositif de chauffage (14).

8. Dispositif de vaporisation selon une quelconque revendication précédente, comprenant également une chambre secondaire (440) pouvant être reliée fluidiquement à la chambre de pression (4) par le biais de la sortie d'écoulement obstruée (12), et de préférence la chambre secondaire comprend une soupape d'entrée (1344) à travers laquelle le gaz peut entrer dans la chambre secondaire et un embout buccal (1346) à travers lequel le fluide peut sortir de la chambre secondaire.

9. Dispositif de vaporisation selon une quelconque revendication précédente, dans lequel la chambre de pression (4) comprend une soupape interne (616) mobile entre une configuration ouverte et une configuration fermée, de telle sorte que lorsque la soupape interne est en position ouverte, la vapeur (32) peut s'évacuer de la chambre de pression à travers la sortie d'écoulement obstruée (12), et lorsque la soupape interne est en position fermée, la vapeur est empêchée de s'évacuer de la chambre de pression à travers la sortie d'écoulement obstruée.

10. Dispositif de vaporisation selon l'une quelconque des revendications 1 à 8, comprenant également une chambre de sortie (718) reliée fluidiquement à la chambre de pression (4) par le biais de la sortie d'écoulement obstruée (12), dans lequel la chambre de sortie comprend une soupape externe (719) mobile entre une configuration ouverte et une configuration fermée, de telle sorte que lorsque la soupape externe est en position ouverte, la vapeur peut s'évacuer de la chambre de sortie à travers la soupape externe, et lorsque la soupape externe est en position fermée, la vapeur est empêchée de s'évacuer de la chambre de sortie à travers la soupape externe.

11. Dispositif de vaporisation selon une quelconque revendication précédente, dans lequel le réservoir (30) de matériau volatil est amovible de la chambre de pression (4) et est remplaçable ou rechargeable, ou dans lequel le réservoir de matériau volatil est solidaire de la chambre de pression et est rechargeable.

12. Procédé de vaporisation d'un matériau volatil dans une chambre de pression (4) comprenant un réservoir (30) de matériau volatil, un dispositif de chauffage (14) et une sortie d'écoulement obstruée (12), comprenant les étapes :

de mesure d'une pression interne dans la chambre de pression ; et,
de commande du dispositif de chauffage en fonction de la pression interne mesurée, **caractérisé en ce que** le dispositif de chauffage est commandé pour amener la vaporisation de matériau volatil pour que la pression interne soit suffisamment élevée pour que la vapeur sortant de la chambre de pression par la sortie d'écoulement obstrué le fasse dans des conditions d'écoulement obstrué.

13. Procédé selon la revendication 12, dans lequel l'étape de commande du dispositif de chauffage (14) comprend la fourniture d'une tension variable au dispositif de chauffage.

**14.** Procédé selon la revendication 12 ou 13, dans lequel le matériau volatil comprend une pluralité de composants de matériau volatil et le procédé comprend également l'étape consistant à amener un rapport prédéterminé de concentrations de composants de matériau volatil en phase vapeur en commandant le dispositif de chauffage (14).

**15.** Procédé selon la revendication 14, dans lequel la pluralité de composants de matériau volatil sont immiscibles, ou dans lequel la pluralité de composants de matériau volatil sont miscibles et s'écartent de la loi de Raoult.

*FIG. 1*

Temperature and density of water vapour at phase boundary

201

202

Density — 
Temperature - - -

Absolute pressure (kPa)

Density (g/l)

Temperature (K)

*FIG. 2*

Isobaric flow through outlet in choked condition

FIG. 3

EP 4 225 087 B1

*FIG. 4*

FIG. 5

FIG. 6

EP 4 225 087 B1

*FIG. 7*

EP 4 225 087 B1

Binary fluid system

FIG. 8

FIG. 9

Equilibrium vapour/Liquid composition if ideal mixture at constant pressure

FIG. 10

EP 4 225 087 B1

*FIG. 11*

*FIG. 12*

**FIG. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019099581 A1